# EUROPEAN PATENT SPECIFICATION

(11) **EP 0 562 088 B1**
(45) Date of publication and mention of the grant of the patent: **25.03.1998**
(21) Application number: 92921750.3
(22) Date of filing: 08.10.1992
(51) Int. Cl.: B32B 25/04, B32B 25/12, C08J 7/04

(54) **RUBBER ARTICLES HAVING A MODIFIED POLYMER LAYER OF ETHER AND ESTER UNITS**
GEGENSTÄNDE AUS GUMMI MIT MODIFIZIERTER POLYMERSCHICHT AUS ETHER- UND ESTERGRUPPEN
ARTICLES EN CAOUTCHOUC AYANT UNE COUCHE POLYMERE MODIFIEE D'UNITES D'ETHER ET D'ESTER

(30) Priority: 11.10.1991 US 776084
(43) Date of publication of application: 29.09.1993
(73) Proprietor: ANSELL PERRY INC., Massillon, OH 44646 (US)
(72) Inventor: NILE, Jeffery, G., Alliance, OH 44601 (US); GROMELSKI, Stanley, J., Canton, OH 44718 (US); BRAIN, Alan, A., York, YO4 5BS (GB); HARDWICK, Steven, T., York, Y01 3NN (GB)
(74) Representative: Bauer, Robert, Dipl.-Ing.
(86) International application number: US9208575
(87) International publication number: WO9306996

(56) References cited:
- DE-A- 2 230 435
- US-A- 3 879 496
- US-A- 4 330 597
- US-A- 4 879 348

## Description

This invention relates to body contacting articles fabricated from natural or synthetic elastomeric materials e.g. natural or synthetic rubbers or polyurethanes. More particularly, the invention relates to medicinal gloves and especially to surgeon's gloves.

In order to improve the donning properties of gloves such as thin rubber or polyurethane gloves of the type used in medical examinations or surgical procedures, it has been customary to incorporate a donning aid at least at the hand contacting surface of the glove. Conventional donning aids have been powders such as talc or starch. However, the use of such particulate donning aids has the disadvantage that particles may drop off the glove into the wound possibly resulting in a granuloma. In order to avoid the use of donning powders other attempts to improve the donning properties of the glove have included the treatment of the hand contacting surface of the glove by halogenation or by lamination with another material having better donning properties than the base rubber. Other articles of natural or synthetic elastomeric materials also suffer problems associated with application due to the frictional resistance of the natural or synthetic elastomeric material employed in the manufacture of the article and the body portion with which it is to be contacted.

Delamination caused during stretching or flexing of the glove has been a problem associated with such laminated gloves particularly when the glove is donned when the hands are wet.

Attempts to improve resistance to delamination have included treatment of the natural or synthetic elastomeric base material surface for example by acid priming so that the laminated material keys well to the base material and remains adhered whilst the base material is stressed. In US Patent No. 4499154 there is disclosed a process for producing a flexible rubber article in which the formed rubber article is subjected to an acid priming step and a neutralising step prior to a polymer coating step.

DE-A-2230435 discloses rubber articles that have a synthetic resin slip coating obtained by contacting the rubber article with a vinyl chloride-alkyl acrylate copolymer or chloride-butyl acrylate copolymer.

The present invention seeks to mitigate the disadvantages of the prior art and to provide body contacting articles such as surgeon's gloves having good donning properties, by using polymers which can be applied directly to the natural or synthetic elastomeric surface without the necessity of pretreating that surface.

Thus the present invention provides a process which is simpler and more economical than those hitherto known.

According to the present invention there is provided a body contacting article which comprises a first layer of a natural or synthetic elastomer and thereon a layer of a polymer comprising repeating units of the formula: 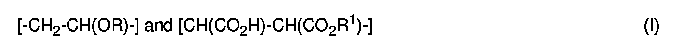 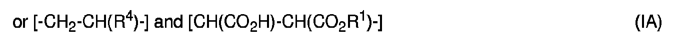 wherein R is a lower alkyl group and R¹ is a lower alkyl group or a (lower) alkoxy (lower) alkyl group, and R⁴ is hydrogen or a phenyl group.

Aptly R is a lower alkyl group containing up to 4 carbon atoms. More suitably R is a methyl group. Aptly R¹ is a lower alkyl group containing up to 9 carbon atoms (e.g. methyl, propyl, butyl, pentyl, hexyl, octyl or nonyl) more aptly up to 4 carbon atoms; or an alkoxy alkyl group having up to 4 carbon atoms in the alkoxy part and up to 4 carbon atoms in the alkyl part (eg. a propoxyethyl group). Suitably, when R is a methyl group, R¹ is a propyl or butyl group. More suitably R¹ is a -(CH₂)₃CH₃ group.

The natural or synthetic elastomer preferably comprises a natural rubber or a polychloroprene (e.g. Neoprene), a nitrile rubber, a styrene butadiene rubber or a polyurethane.

The polymers for use in the polymer layer favourably comprise or consist essentially of repeating units of formula (I) or (IA). Such polymers may be referred to as copolymers of a vinyl alkyl ether and a maleic ester (I), or of an alkylene and a maleic ester (IA).

Preferably the body contacting article is a surgeon's glove.

According to a further embodiment of the invention there is also provided a surgeon's glove which comprises a rubber glove which has on the hand contacting surface thereof a layer of a polymer comprising repeating units of the formula: 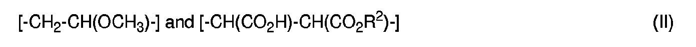 wherein R² is lower alkyl group. Suitably R² is an alkyl group containing up to 9 carbon atoms, more suitably up to 4 carbon atoms. Preferably R² is a propyl or butyl group. More preferably R² is an n-butyl or a -(CH₂)₃CH₃ group.

The preferred polymers for use in the polymer layer favourably comprise or consist essentially of repeating units of the formula II. Such polymers may be referred to as copolymers of vinyl methyl ether and a maleic ester.

Polymers having repeating units of formula I or II are commercially available and are sold by GAF Corporation under their trademark GANTREZ. Apt polymers are those of the GANTREZ ES series of resins and are described as alkyl monoesters of poly(methyl vinyl ether - maleic acid). Suitable resins of this series are the isoproply and n-butyl esters sold under the trade names ES-335, ES-425 and ES435.

Other polymers may however be suitable and these includes polymers of;
(i) the butyl half ester of poly(ethylene/maleic acid)- which is a rubbery material that laminates satisfactorily to rubber.
(ii) the butyl half ester of Scripset 520 (butyl half ester of poly (Styrene/maleic acid) which is a glassy material.
(iii) SMA 2625 (partly esterified poly(styrene/maleic acid))- which is a glassy material that laminates satisfactorily to rubber.

(Scripset 520 is a trade mark and relates to a substance obtainable from Monsanto).

The polymers having repeating units of formula I, IA or I may be applied directly to formed articles having a natural or synthetic elastomer surface.

Accordingly therefore there is also provided a method for producing a body contacting article comprising a first layer of a natural or synthetic elastomeric material, which method comprises applying to said first layer a layer of a polymer having repeating units of the formula I, IA or II as hereinabove defined.

The method of the present invention may be employed as part of the article manufacturing process. For example, where the article is a glove such as a surgeon's glove, the glove may be first formed by a conventional dipping procedure wherein a suitably shaped former is first dipped into a coagulant and thereafter into a latex solution comprising the natural or synthetic elastomer, e.g. natural rubber from which the article is to be formed. After withdrawal from the dipping bath the article may then be subjected to a conventional washing or leaching step prior to application of the polymer layer.

In a preferred aspect of the process of the present invention the formation of the polymer layer on the natural or synthetic elastomer surface takes place prior to final curing of the elastomer. Thus in a conventional glove dipping process, the process train may be modified by incorporating the process of the invention as an intermediate step between the leaching and curing steps.

Thus in accordance with an embodiment of the invention there is provided a process for the production of dipped articles in which a layer of a polymer having repeating units of formula (I), (IA) or (II) is applied to a natural or synthetic eastomer surface of the article prior to final curing of the rubber article.

Aptly, the polymer may be applied to the elastomer surface as a solution. Suitably the solution employed is an alcoholic solution. Preferred alcohols for forming the polymer solution include alkanols such as ethanol and isopropanol.

The polymer content in such solutions should not be more than 50% by weight of the solution, aptly up to about 15% by weight of the total solution, and preferably up to about 8% by weight. Generally, the polymer contact of the solution will be at least 2% by weight of polymer. Preferably polymer contents of from 4 to 6% by weight may be used.

The elastomers can be obtained as latices, which can then be dipped and coated. Cosolvents can be used in the preparation of the latices and the elastomer may be initially present in an emulsion, e.g. a polyurethane emulsion.

The polymer layer may be formed by conventional solution coating procedures but is aptly formed by dipping the article into a solution of the polymer.

The polymer solution may be employed at temperatures below the boiling range of the polymer solvent and may suitably be used at ambient temperatures.

Although the polymer may be used alone, additional hydroxyl containing compounds may be added to the polymer solution. Aptly such hydroxyl containing compounds may include water or polyhydroxy compounds such as polyethylene glycol.

Aptly water may be present in the polymer solution in amounts up to 50% by weight based upon the weight of the solution.

Preferred polyethylene glycols for inclusion with polymers having repeating units of formula (I), (IA) or (II) are those commercially available from Union Carbide under their trademark CARBOWAX. Polyhydroxy compounds, as typified by the carbowaxes, may be present in amounts up to about 30% by weight of the polymer aptly in amounts up to 10%, possibly up to 5%, up to 1% or up to 0.5% by weight of the polymer.

After application of the polymer layer, the article may be dried and cured according to conventional procedures.

The article may thereafter be subjected to post-curing treatments such as halogenation in order to impart desired physical properties to the product.

We have found that further improvements in the hand donning properties may be obtained by treatment with an agent such as a surfactant. An apt surfactant for use with the invention is cetyl pyridinium chloride (CPC). Although this surfactant may be employed alone, it may suitably be employed as a complex with a long chain acid, more suitably with a fatty acid. Preferred fatty acids include stearic acid. Lauric acid is especially preferred. CPC in admixture with fatty acid derivatives such as lauryl sulphonate may also be used but these mixtures are less preferred.

Suitable complexes may be formed by adding each of the constituents to water and applying the aqueous complex to the article. A preferred complex may be formed by admixing CPC and lauric acid in water and heating such that each constituent is present in amounts of about 2% by weight of the aqueous complex.

The complex may be applied either by spraying it onto the elastomer surface of the article or by immersing the article in the aqueous complex. Suitably the complex is applied at elevated temperature. Spraying is preferred. In a preferred embodiment the spraying is carried out at elevated temperature, aptly at about 54,44°C (130°F). Dipping applications may be carried out at temperatures of about 37,78°C (100°F).

Other donning aids such as silicones used either along or in combination wit the CPC containing complexes may also be used with advantage.

Thereafter the treated article is dried.

Examination of the elastomer surface and the polymer layer thereon, for example by a Scanning Electron Microscope (SEM) shows the polymer to be distributed over the whole of the rubber surface.

The articles of the invention may have flat polymer coatings. However, in preferred form of the invention, the polymer layer, whilst being continuous has raised areas (domains) which are thicker than the surrounding polymer layer. In these preferred forms of the invention the thickness of the raised domains may be up to 25gm thicker than that of the remaining areas, favourably up to about 7pm thicker. The remaining areas may be less than about 8pm thickness, aptly between 6 and 7pm, more aptly less than 1µm

The occurrence of raised domain architecture of the polymer surface depends mainly upon the condition of the elastomer surface to which the polymer is applied.

We have found that if the elastomer surface contains moisture, for example after the leaching stage in a conventional dipping process train, a raised domain architecture will occur when the polymer is applied. Thus it is preferred to apply the polymer between the leaching and curing stages when the articles are produced by a dipping process.

Further improvements in the donning preparation of articles such as gloves when produced in accordance with the invention have been determined when a polymer has repeating units of the formula 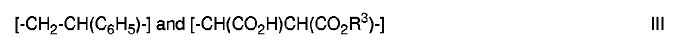 where R³ is an alkyl group. Aptly R³ is a lower alkyl group, more aptly R³ is an alkyl group, containing up to 4 carbon atoms. Suitable polymers having repeating units of formula (III) are sold by Monsanto under their trade name SCRIPTSET. Preferred grades are those sold under the designations SCRIPTSET 540 and SCRIPTSET 550.

Other similar and suitable polymers may be those sold by Atochem under their trademark SMA e.g. the polymer sold under the trade name SMA 2625, which is effective in combination with the polymers having repeating units of formula I and II.

Polymers having repeating units of formula (III) may be incorporated directly into the solution of the polymer having repeating units of formula (I), (IA) or (II). Aptly polymer (III) may present in the polymer (II) solution in an amount of greater than about 5% by weight of the total solids content of the solution. Preferably the concentration of polymer III will be about 20% by weight or less of the solution solids. In favoured form the solution for forming the polymer layer on the article will comprise or consist essentially of 94% by weight of ethanol, 4.8% by weight of a polymer having repeating units of formula (I) or (IA) and 1.2% be weight of a polymer having repeating units of formula (III).

The surface morphology of articles having the above described modified polymer layer shows that the raised areas have further raised areas thereon.

Gloves produced with said modified layers have good donning properties especially wet hand donning properties.

In addition to having the modified polymer layer thereon, the donning properties body contact articles may further improve by the use of donning aids, for example as hereinbefore described.
The invention will now be illustrated by the following examples.

### Example 1

An alcoholic coagulant solution was prepared according to the formulation:

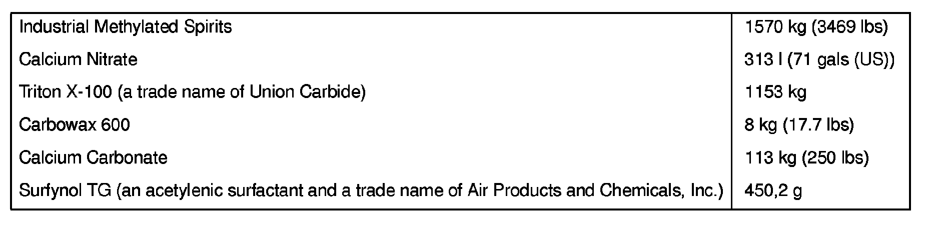

Glove formers, for moulding a surgeon's glove, were immersed in the coagulant solution for 1 minute. The temperature of the coagulated solution was maintained at 43,33°C (110°F).

The coagulant treated former was then dipped into a latex solution for 1 minute. The temperature of the latex was ambient temperature and the solution had the following composition:

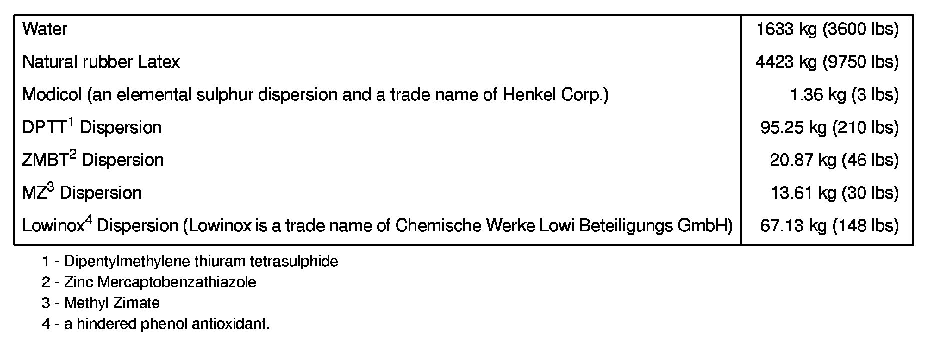

The final solids content of the latex solution was reduced by the addition of a further 362,87 kg (800 lbs) of water.

Upon removal from the latex solution the coated formers are then immersed in a leaching bath, maintained at 71 °C (160°F), for 2 minutes.

The leached coated formers were immersed in a polymer bath, maintained at ambient temperature before passage to a curing oven where they were dried at 115.6°C (240°F) for 20 minutes.

The polymer solution was prepared by diluting an ethanolic solution of GANTREZ ES-425 copolymer to 4% by weight solids content with industrial methylated spirits. GANTREZ ES-425 has repeating units of Formula (II) in which R² is a (CH₂)₃CH₃ group. To the 4% resin solution was added a polyethylene glycol (Carbowax 600) in an amount of 20% by weight based on the weight of the resin.

After curing all the gloves were removed from the formers and subjected to a conventional halogenation procedure and then sprayed with a donning aid consisting of:

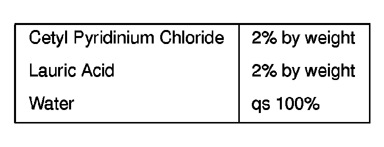

The temperature of the spraying solution was 37.8°C (100°F). The gloves were then dried a 60°C (140°F) for 5 minutes.

The thus produced gloves exhibit good hand donning properties.

### Examples 2-5

Example 1 was repeated except that the polymer solution consisted essentially of a 4% solution of GANTREZ ES 425 in industrial methylated spritis. The thus treated polymer gloves were subjected to the post treatments summarised in the following table.

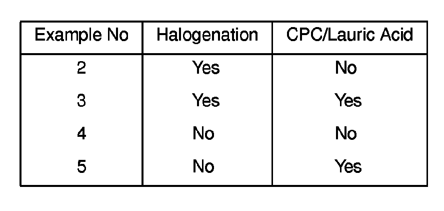

The thus produced gloves exhibit good hand donning properties.

### Example 6

Examples 2-5 were repeated except that the polymer solution consisted of:

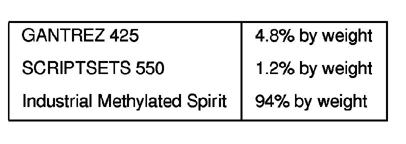

The gloves thus produced exhibited good wet and dry hand donning properties.

The surface morphology of gloves produced in accordance with Examples 3 and 6 was examined together with that of a control sample which is a conventional halogenated glove having no polymer treatment in accordance with the invention.

Surface roughness measurements and surface traces were obtained by surface profilometry using a Rank Taylor-Hobson TALLYSURF 10 profilometer (TALLYSURF is a trade name of The Rank Organization Ltd.).

Glove samples were taken from the middle finger of each glove for the control samples and the glove of Example 3 the amplification (Vᵥ) was x5000 whereas (because of the increase in difference between the lowest and highest points of the probe) that for the glove for Example 6 was Vᵥ = X1000. The Vₕ setting was 0.8mm (cut-off) in each case.

The surface roughness (averaged in three readings) was

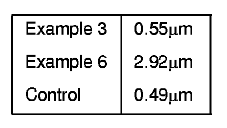

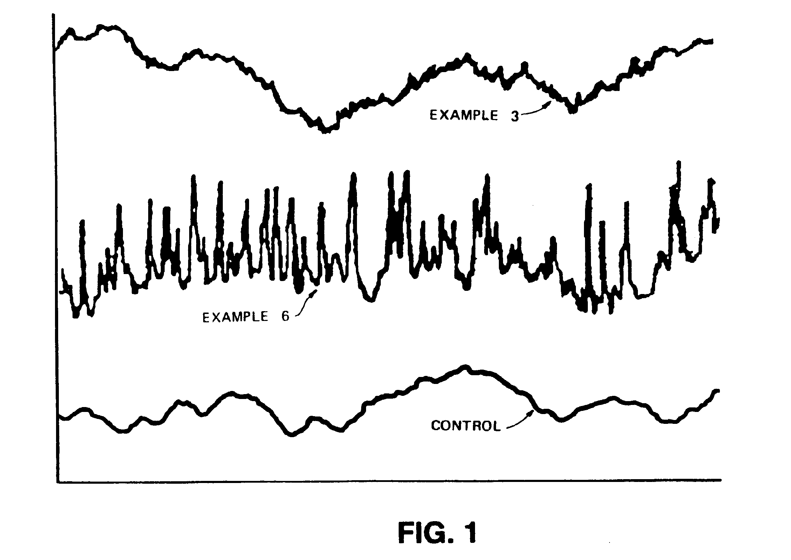

Fig. 1 in the accompanying drawing depicts the surface trace for each glove over a length of 3cm (Vₕ - x10 and Vᵥ - x1000).

### Example 7

### The Application of Gantrez E5-425/Scripset 540 Coatinas to Synthetic Latices

The synthetic latices shown in table 1 below, were coagulation dipped, as described below, and coated with a solution (an industrial Methylated Spirits) of 2.8% by weight GANTREZ ES-425, 1.2% by weight SCRIPTSET 540 and 0.8% by weight CARBOWAX 600 (referred to as 70/30 blend of Gantrez ES-425 and Scripset 540).

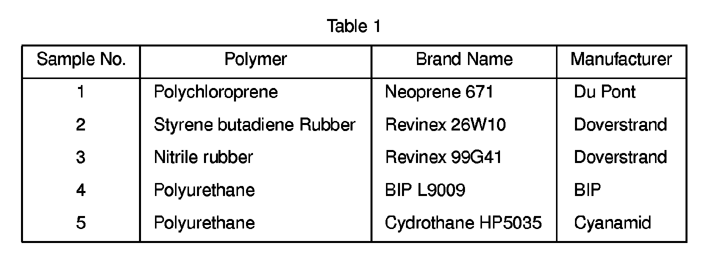

Preheated (to 115°C) ceramic glove formers were used to prepare coated latex gloves using the steps shown in Table 2. All the dipping operations were carried out by hand.

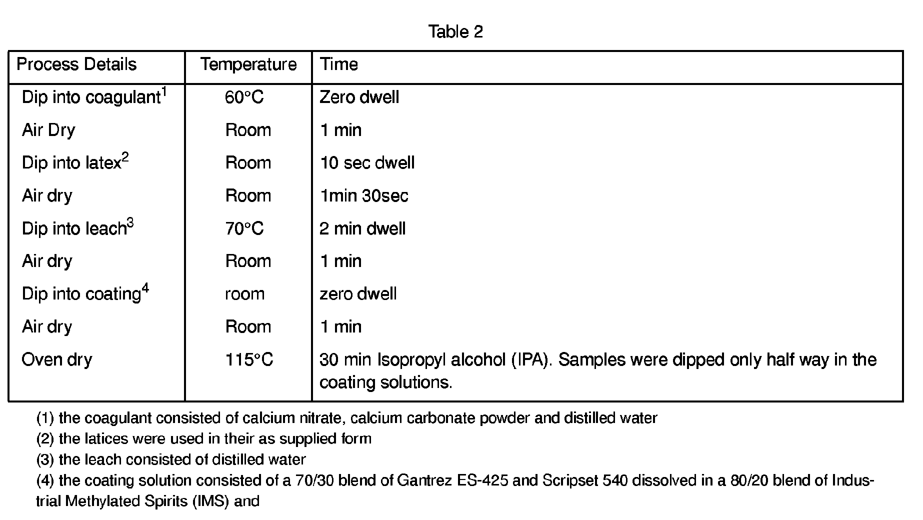

Both the coated and uncoated latex surfaces were examined using optical microscopy. This revealed that roughened coatings were present on the synthetic latices. These roughened coatings, give rise to good damp hand donning performances.

### Example 8

### The Application of a Range of Partly Esterified Maleic Anhydride Derived Polymers to Natural Rubber (NR) Latex

The polymers listed below were applied onto coagulation dipped natural rubber latex surfaces.
1. Gantrez ES 225 (Acid primed latex)
2. Isopropyl half ester of Gantrez AN 169
3. Propoxyethyl half ester of Gantrez AN169
4. n-Hexyl half ester of Gantrez AN169
5. n-Nonyl half ester of Gantrez AN169
6. n-Butyl half ester of polyethylene/maleic acid
7. SMA 2625

Preheated (to 115°C) ceramic glove former were used to prepare samples using the procedure set out in Table 3 below. The dipping operations were carried out on the Cotswold Dipping Machine and Glove Coating Rig.

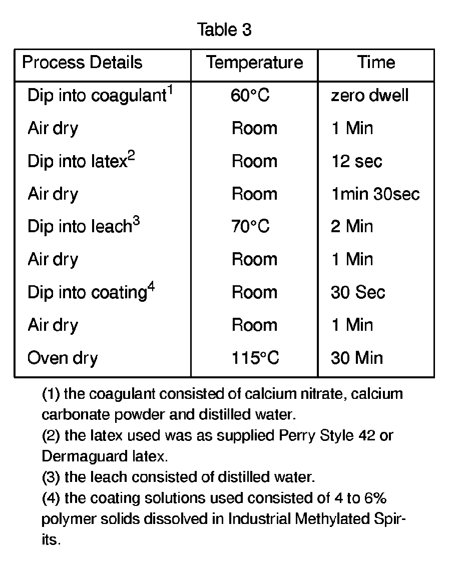

Satisfactory hand donning characteristic results were obtained with the isopropyl half ester of Gantrez AN 169 and with the n-butyl half ester of polyethylene/maleic acid. The other materials gave even better results

### Example 9

A glove former for moulding a surgeon's glove was dipped into WITCO # A 127-71 polyurethane without coagulant (WITCO is a trade name of Witco). The resulting film was dried for 5 mins at 130°F and then overdipped with an overdip formulation, comprising:-
2.8% Gantrez ES-425
1.2% Scriptset 540
0.8% Carbowax 600

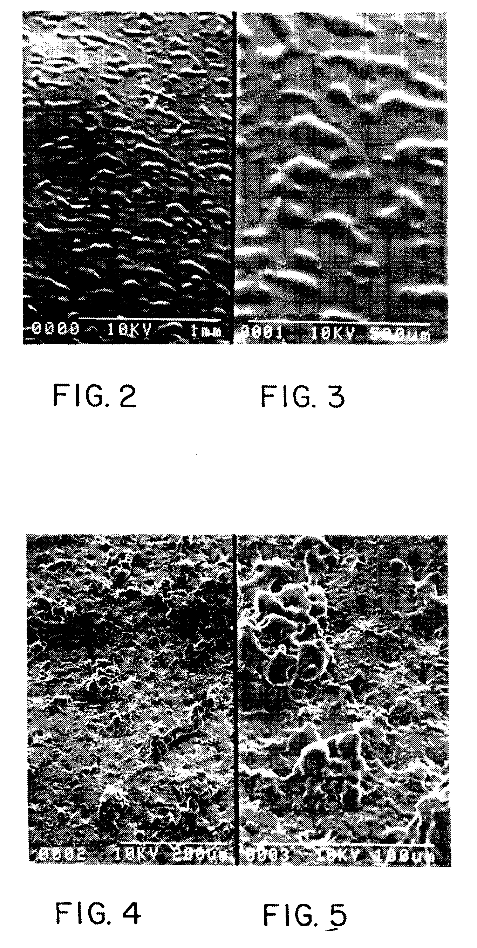

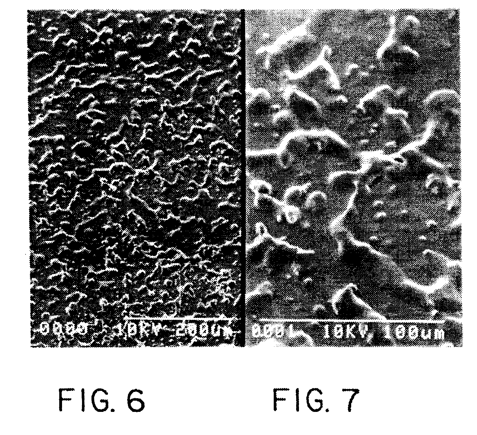

Examination of the Gantrez-Scriptset surface by Scanning electron microscope was carried out and the results are shown in Figures 2 (40 x magnification) and 3 (100 x magnification), from which is can be seen that domains were formed. The glove was found to exhibit good hand donning properties.

### Example 10

The process described in Example 9 was repeated, but using Du Pont Neoprene 571 latex instead of polyurethane and including an extra step of dipping the glove former in coagulant and drying it before dipping into the Neoprene latex.

Examination of the Gantrez-Scriptset surface by Scanning electron microscope was carried out and the results are shown in Figures 4 (150 x magnification) and 5 (500 x magnification), from which is can be seen that domains were formed. The glove was found to exhibit good hand donning properties.

### Example 11

The process described in Example 9 was repeated but using a latex prepared from a nitrile sold by Reichhold Chemicals Inc. under their trade name TYLAC 68-060-00 instead of polyurethane and using 12 dips into the latex instead of 1. A further difference was that after drying the nitrile coating and before overdipping the sample was leached at 71 °C (160°F) for 2 mins.

Examination of the Gantrez-Scriptset surface by Scanning electron microscope was carried out and the results are shown in Figures 6 (150 x magnification) and 7 (500 x magnification), from which is can be seen that domains were formed. The glove was found to exhibit good hand donning properties.

Additional detail of some of the abovementioned coating polymers is given below:-
Gantrez ES-225 and Half Esters of Gantrez AN169 are believed to contain repeating units of the formula:- 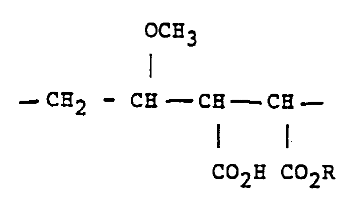
   where R = ethyl (ie. Gantrez ES-225)
   R = isopropyl
   R = Propoxyethyl
   R = n-pentyl for the other half esters
   R = n-hexyl
   R = n-octyl
   R = n-nonyl
n-Butyl Half Ester of Poly/ethylene/maleic acid is believed to contain repeating units of the formula:- 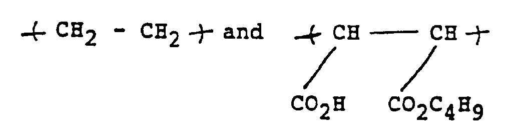
Scripset 540 and 550 and Butylated Scripset 520 are believed to contain repeating units of the formula:- 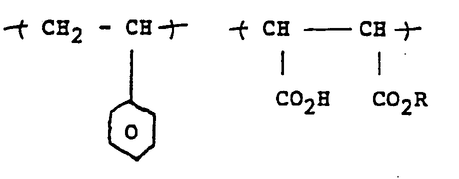
   where R = n-butyl (for Scripset 540)
   R = ethylln-butyl mixture (for Scripset 550)
   R = n-butyl (for Butylated Scripset 520)

   for Butylated Scripset 520 the ratio of the number of the first units to the number of the second units is 1:1, Scripsets
   540 and 550 the ratio is 1:<1.
   SMA 2625
   is believed to be 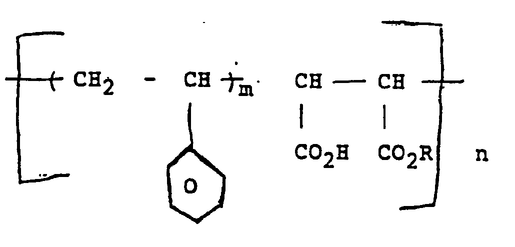
   where m = 1 to 3
   n=₆to8
   where R is a low alkyl group.

## Claims

1. A body contacting article which comprises a first layer of a natural or synthetic elastomer and thereupon a layer of polymer comprising repeating units of the formula 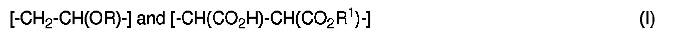 or 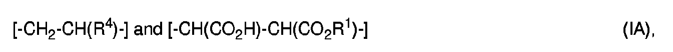 wherein R is a lower alkyl group and R¹ is a lower alkyl group or a (lower) alkoxy (low) alkyl group, and R⁴ is hydrogen or a phenyl group.

2. An article according to claim 1, wherein R is a methyl group.

3. An article according to claim 2, wherein R¹ is a propyl or butyl group.

4. An article according to claim 2, wherein R¹ is a -(CH₂)₃CH₃ group.

5. An article according to any one of claims 1 to 4, wherein said polymer layer comprises a polymer having repeating units of formula (I) or (IA), wherein R and R¹ are as defined in any one of claims 1 to 4 and a polymer having repeating units of the fomula 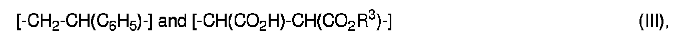 wherein R³ is an alkyl group.

6. An article according to any one of the preceding claims, wherein said elastomer comprises natural rubber, a polychloroprene, a nitrile rubber, a styrene-butadiene rubber or a polyurethane.

7. A body contacting article which comprises a first layer of rubber and thereupon a layer of polymer comprising repeating units of the formula 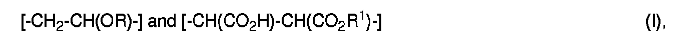 wherein R is a lower alkyl group and R¹ is a lower alkyl group.

8. An article as claimed in any one of claims 1 to 7, which comprises a surgeon's glove.

9. A surgeon's glove which comprises a glove comprising a natural or synthetic elastomer, which has on the hand contacting surface thereof a layer of polymer comprising repeating units of the formula 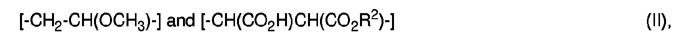 wherein R² is a lower alkyl group.

10. A glove according to claim 9 wherein R² is a -(CH₂)₃CH₃ group.

11. A glove according to claim 9 or 10, wherein said polymer layer comprises a polymer having repeating units of formula (II) wherein R and R¹ are as defined in claim 1 and a polymer having repeating units of a polymer of formula (III) where R³ is as defined in claim 5.

12. A glove according to any one of claims 9 to 11, wherein said elastomer comprises natural rubber, a polychloroprene, a nitrile rubber, a styrene butadiene rubber or a polyurethane.

13. A surgeon's glove according to any one of claims 9 to 12, wherein the polymer layer is continuous and has raised areas.

14. A surgeon's glove which comprises a rubber glove which has on the hand contacting surface thereof a layer of polymer comprising repeating units of the formula 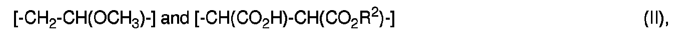 wherein R² is a lower alkyl group.

15. A method for production of a body contacting article which article comprises a first layer of a natural or synthetic elastomer which method comprises forming a layer of a polymer comprising repeating units of the formula 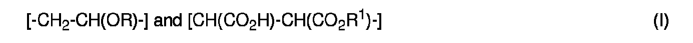 or 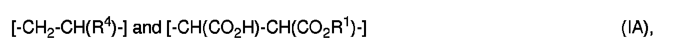 wherein R, R¹ and R⁴ are as defined in any one of claims 1 to 4, upon said first layer of natural or synthetic elastomer.

16. A method according to claim 15, wherein the first layer is derived from a latex.

17. A method according to claim 15 or 16, wherein the polymer is applied as a solution in ethanol or isopropanol.

18. A method according to claim 17, wherein the polymer content is from 4 to 6% by weight of the polymer solution.

19. A method accoording to claim 17 or 18, wherein the polymer solution contains at least one other hydroxyl compound selected from water and a polyethylene glycol.

20. A method according to any one of claims 15 to 19, wherein the polymer further comprises a polymer having repeating units of the formula 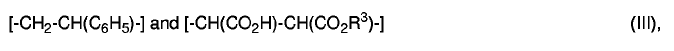 wherein R³ is an alkyl group.

21. A method according to claim 20, wherein the polymer comprises up to 20% by weight of a polymer having repeating units of formula (III).

22. A method according to any one of claims 15 to 21, wherein the polymer layer is treated with a surfactant comprising a mixture of cetyl pyridinium chloride and lauric acid.

23. A method according to any one of claims 15 to 22, wherein the elastomer comprises natural rubber, a polychloroprene, a nitrile rubber, a styrene butadiene rubber or a polyurethane.

24. A method for production of a body contacting article which article comprises a first layer of rubber which method comprises forming a layer of a polymer comprising repeating units of the formula 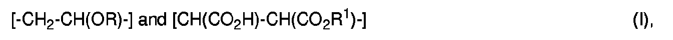 wherein R is a lower alkyl group and R¹ is a lower alkyl group, upon said first layer of rubber.

## Patentansprüche

1. Körperkontaktartikel, der eine erste Schicht eines natürlichen oder synthetischen Elastomers und darauf eine Schicht eines Polymers umfaßt, das sich wiederholende Einheiten der Formel 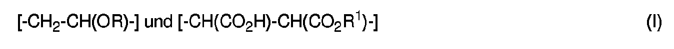 oder 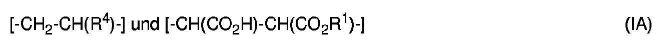 enthält, worin R eine Niederalkylgruppe ist und R¹ eine Niederalkylgruppe oder eine (Nieder-)Alkoxy-(nieder- )alkylgruppe ist und R⁴ ein Wasserstoffatom oder eine Phenylgruppe ist.

2. Artikel nach Anspruch 1, worin R eine Methylgruppe ist.

3. Artikel nach Anspruch 2, worin R¹ eine Propyl- oder Butylgruppe ist.

4. Artikel nach Anspruch 2, worin R¹ eine -(CH₂)₃CH₃-Gruppe ist.

5. Artikel nach einem der Ansprüche 1 bis 4, worin die Polymerschicht ein Polymer mit sich wiederholenden Einheiten der Formel (I) oder (IA), worin R und R¹ wie in einem der Ansprüche 1 bis 4 definiert sind, und ein Polymer mit sich wiederholenden Einheiten der Formel 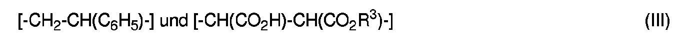 umfaßt, worin R³ eine Alkylgruppe ist.

6. Artikel nach einem der vorstehenden Ansprüche, worin das Elastomer natürlichen Katschuk, ein Polychloropren, einen Nitrilkautschuk, einen Styrol-Butadienkautschuk oder ein Polyurethan enthält.

7. Körperkontaktartikel, der eine erste Schicht eines Kautschuks und darauf eine Schicht eines Polymers umfaßt, das sich wiederholende Einheiten der Formel 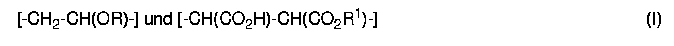 enthält, worin R eine Niederalkylgruppe ist und R¹ eine Niederalkylgruppe ist.

8. Artikel nach einem der Ansprüche 1 bis 7, der einen Chirurgenhandschuh umfaßt.

9. Chirurgenhandschuh, der einen Handschuh umfaßt, welcher ein natürliches oder synthetisches Elastomer enthält, das auf seiner Handkontaktoberfläche eine Schicht eines Polymers aufweist, das sich wiederholende Einheiten der Formel 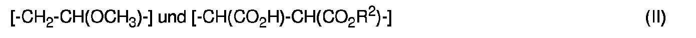 enthält, worin R² eine Niederalkylgruppe ist.

10. Handschuh nach Anspruch 9, worin R² eine -(CH₂)₃CH₃ Gruppe ist.

11. Handschuh nach Anspruch 9 oder 10, worin die Polymerschicht ein Polymer mit sich wiederholenden Einheiten der Formel (11), worin R und R¹ wie in Anspruch 1 definiert sind, und ein Polymer mit sich wiederholenden Einheiten eines Polymers der Formel (111) umfaßt, worin R³ wie in Anspruch 5 definiert ist.

12. Handschuh nach einem der Ansprüche 9 bis 11, worin das Elastomer Naturkautschuk, ein Polychloropren, einen Nitrilkautschuk, einen Styrolbutadienkautschuk oder ein Polyurethan enthält.

13. Chirurgenhandschuh nach einem der Ansprüche 9 bis 12, worin die Polymerschicht kontinuierlich ist und erhöhte Bereiche aufweist.

14. Chirurgenhandschuh, der einen Kautschukhandschuh umfaßt, welcher auf seiner Handkontaktoberfläche eine Schicht eines Polymers mit sich wiederholenden Einheiten der Formel 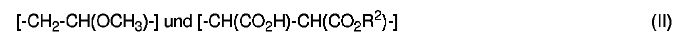 aufweist, worin R² eine Niederalkylgruppe ist.

15. Verfahren zur Herstellung eines Körperkontaktartikels mit einer ersten Schicht aus einem natürlichen oder synthetischen Elastomer, welches Verfahren das Bilden einer Schicht eines Polymers mit sich wiederholende Einheiten der Formel 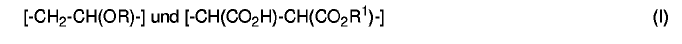 oder 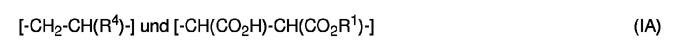 auf der ersten Schicht des natürlichen oder synthetischen Elastomers umfaßt, worin R, R¹ und R⁴ wie in einem der Ansprüche 1 bis 4 definiert sind.

16. Verfahren nach Anspruch 15, worin die erste Schicht von einem Latex abgeleitet ist.

17. Verfahren nach Anspruch 15 oder 16, worin das Polymer als eine Lösung in Ethanol oder Isopropanol aufgebracht wird.

18. Verfahren nach Anspruch 17, worin der Polymergehalt von 4 bis 6 Gew.-% der Polymerlösung beträgt.

19. Verfahren nach Anspruch 17 oder 18, worin die Polymerlösung mindestens eine andere Hydroxylverbindung enthält, die aus Wasser und einem Polyethylenglykol ausgewählt wird.

20. Verfahren nach einem der Ansprüche 15 bis 19, worin das Polymer weiter ein Polymer mit sich wiederholenden Einheiten der Formel 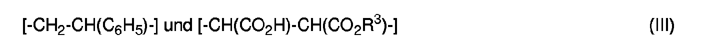 enthält, worin R³ eine Alkylgruppe ist.

21. Verfahren nach Anspruch 20, worin das Polymer bis zu 20 Gew.-% eines Polymers mit sich wiederholenden Einheiten der Formel (111) umfaßt.

22. Verfahren nach einem der Ansprüche 15 bis 21, worin die Polymerschicht mit einem grenzflächenaktiven Mittel behandelt wird, das ein Gemisch aus Cetylpyridiniumchlorid und Laurinsäure umfaßt.

23. Verfahren nach einem der Ansprüche 15 bis 22, worin das Elastomer Naturkautschuk, ein Polychloropren, einen Nitrilkautschuk, einen Styrol-Butadienkautschuk oder ein Polyurethan umfaßt.

24. Verfahren zur Herstellung eines Körperkontaktartikels mit einer ersten Schicht eines Kautschuks, welches Verfahren das Bilden einer Schicht eines Polymers mit sich wiederholenden Einheiten der Formel 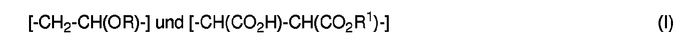 auf der ersten Kautschukschicht umfaßt, worin R eine Niederalkylgruppe und R¹ eine Niederalkylgruppe ist.

## Revendications

1. Article pour contact corporel qui comprend une première couche d'un élastomère naturel ou synthétique et, par-dessus, une couche de polymère comprenant des motifs répétitifs de formule : 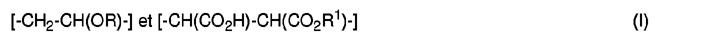 ou 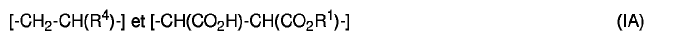 où R est un groupe alkyle inférieur et R¹ est un groupe alkyle inférieur ou un groupe alkoxy (inférieur)-alkyle (inférieur), et R⁴ est l'hydrogène ou un groupe phényle.

2. Article selon la revendication 1, dans lequel R est un groupe méthyle.

3. Article selon la revendication 2, dans lequel R¹ est un groupe propyle ou butyle.

4. Article selon la revendication 2, dans lequel R¹ est un groupe -(CH₂)₃cH₃.

5. Article selon l'une quelconque des revendications 1 à 4, dans lequel ladite couche de polymère comprend un polymère ayant des motifs répétitifs de formule (I) ou (IA) dans laquelle R et R¹ sont tels que définis dans l'une quelconque des revendications 1 à 4, et un polymère ayant des motifs répétitifs de formule : 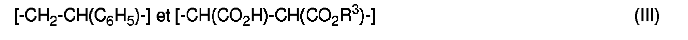 dans laquelle R³ est un groupe alkyle.

6. Article selon l'une quelconque des revendications précédentes, dans lequel ledit élastomère comprend du caoutchouc naturel, un polychloroprène, un caoutchouc de nitrile, un caoutchouc styrènelbutadiène ou un polyuréthane.

7. Article pour contact corporel qui comprend une première couche de caoutchouc et, par-dessus, une couche de polymère comprenant des motifs répétitifs de formule : 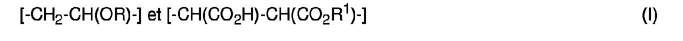 dans laquelle R est un groupe alkyle inférieur et R¹ est un groupe alkyle inférieur.

8. Article selon l'une quelconque des revendications 1 à 7, qui comprend un gant de chirurgien.

9. Gant de chirurgien qui comprend un gant comprenant un élastomère naturel ou synthétique, qui a, sur sa surface en contact avec la main, une couche de polymère comprenant des motifs répétitifs de formule : 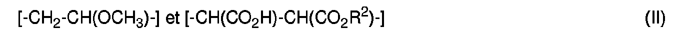 dans laquelle R² est un groupe alkyle inférieur.

10. Gant selon la revendication 9, dans lequel R² est un groupe -(CH₂)₃CH₃-

11. Gant selon la revendication 9 ou 10, dans lequel ladite couche de polymère comprend un polymère ayant des motifs répétitifs de formule (II) dans laquelle R et R¹ sont tels que définis dans la revendication 1 et un polymère ayant des motifs répétitifs d'un polymère de formule (III) dans laquelle R³ est tel que défini dans la revendication 5.

12. Gant selon l'une quelconque des revendications 9 à 11, dans lequel ledit élastomère comprend du caoutchouc naturel, un polychloroprène, un caoutchouc de nitrile, un caoutchouc styrène/butadiène ou un polyuréthane.

13. Gant de chirurgien selon l'une quelconque des revendications 9 à 12, dans lequel la couche de polymère est continue et a des zones saillantes.

14. Gant de chirurgien qui comprend un gant en caoutchouc qui a, sur sa surface en contact avec la main, une couche de polymère comprenant des motifs répétitifs de formule : 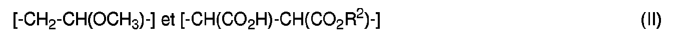 dans laquelle R² est un groupe alkyle inférieur.

15. Procédé pour la production d'un article pour contact corporel, cet article comprenant une première couche d'un élastomère naturel ou synthétique, ce procédé comprenant la formation d'une couche d'un polymère comprenant des motifs répétitifs de formule : 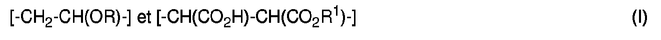 ou 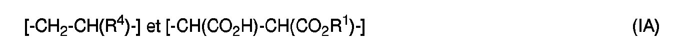 où R, R¹ et R⁴ sont tels que définis dans l'une quelconque des revendications 1 à 4,
au-dessus de ladite première couche d'élastomère naturel ou synthétique.

16. Procédé selon la revendication 15, dans lequel la première couche est dérive d'un latex.

17. Procédé selon la revendication 15 ou 16, dans lequel le polymère est appliqué sous la forme d'une solution dans l'éthanol ou l'isopropanol.

18. Procédé selon la revendication 17, dans lequel la teneur en polymère est de 4 à 6 % en poids de la solution de polymère.

19. Procédé selon la revendication 17 ou 18, dans lequel la solution de polymère contient au moins un autre composé hydroxylé choisi parmi l'eau et un polyéthylèneglycol.

20. Procédé selon l'une quelconque des revendications 15 à 19, dans lequel le polymère comprend en outre un polymère ayant des motifs répétitifs de formule : 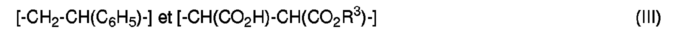 dans laquelle R³ est un groupe alkyle.

21. Procédé selon la revendication 20, dans lequel le polymère comprend jusqu'à 20 % en poids d'un polymère ayant des motifs répétitifs de formule (III).

22. Procédé selon l'une quelconque des revendications 15 à 21, dans lequel la couche de polymère est traitée avec un tensioactif comprenant un mélange de chlorure de cétylpyridinium et d'acide laurique.

23. Procédé selon l'une quelconque des revendications 15 à 22, dans lequel l'élastomère comprend du caoutchouc naturel, un polychloroprène, un caoutchouc de nitrile, un caoutchouc styrène/butadiène ou un polyuréthane.

24. Procédé pour la production d'un article pour contact corporel, cet article comprenant une première couche de caoutchouc, ce procédé comprenant la formation d'une couche d'un polymère comprenant des motifs répétitifs de formule : 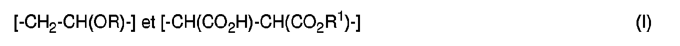 dans laquelle R est un groupe alkyle inférieur et R¹ est un groupe alkyle inférieur, au-dessus de ladite première couche de caoutchouc.
